# EUROPÄISCHE PATENTSCHRIFT

(11) **EP 1 758 498 B1**
(45) Veröffentlichungstag und Bekanntmachung des Hinweises auf die Patenterteilung: **15.12.2010**
(21) Anmeldenummer: 05760833.3
(22) Anmeldetag: 23.06.2005
(51) Int. Cl.: A61B 3/113, A61F 9/008

(54) **VORRICHTUNG UND VERFAHREN ZUM ERFASSEN DER RÄUMLICHEN LAGE DER OPTISCHEN ACHSE EINES AUGES**
PROCESS AND DEVICE FOR SENSING THE POSITION IN SPACE OF THE OPTICAL AXIS OF AN EYE
DISPOSITIF ET PROCEDE DE DETECTION DE LA POSITION SPATIALE DE L'AXE OPTIQUE D'UN OEIL

(30) Priorität: 25.06.2004 DE 102004030904
(43) Veröffentlichungstag der Anmeldung: 07.03.2007
(73) Patentinhaber: Neuhann, Thomas, 80801 München (DE); Hassel, Jörg, 82284 Grafrath (DE)
(72) Erfinder: HASSEL, Jörg, M., 82284 Grafrath (DE)
(74) Vertreter: Rösler, Uwe
(86) Internationale Anmeldenummer: PCT/EP2005/006824
(87) Internationale Veröffentlichungsnummer: WO 2006/000423

(56) Entgegenhaltungen:
- EP-A- 1 209 553
- WO-A-01/45606
- US-A- 4 729 652
- US-A1- 2002 198 516
- CRANE H D ET AL: "GENERATION-V DUAL-PURKINJE-IMAGE EYETRACKER" APPLIED OPTICS, OPTICAL SOCIETY OF AMERICA,WASHINGTON, US, Bd. 24, Nr. 4, 15. Februar 1985 (1985-02-15), Seiten 527-537, XP001181493 ISSN: 0003-6935

## Beschreibung

### Technisches Gebiet

Die Erfindung bezieht sich auf eine Vorrichtung und ein Verfahren zum Erfassen der räumlichen Lage der optischen Achse des Auges eines menschlichen oder tierischen Probanden sowie zum Zentrieren eines Bezugssystems relativ zur optischen Achse, mit wenigstens einer ein Parallellichtstrahlenbündel emittierenden Lichtquelle, einem gegenüber der Lichtquelle vorgesehenen Positionsbereich für den Probanden, Mittel zur relativen Lageausrichtung des Parallelstrahlenlichtbündels gegenüber dem Auge des Probanden sowie wenigstens einer Detektoreinheit zur Detektion von in und am Auge durch das Parallellichtstrahlenbündel hervorgerufenen Reflexionsereignissen.

### Stand der Technik

Vorrichtungen der vorstehend beschriebene Gattung werden vornehmlich auf dem Gebiet der Augenheilkunde, insbesondere zur Korrektur der Fehlsichtigkeit eines Auges unter Verwendung eines Therapielasersystems, insbesondere eines Excimerlasers, eingesetzt, mit dem eine gezielte Materialabtragung an oder innerhalb der Cornea durchgeführt wird, um auf diese Weise eine erwünschte Änderung an der Hornhautkurvatur und eine damit verbundene Korrektur der optischen Wirkung der Cornea zur erzielen.

Die sog. photorefraktive Korrektur von Fehlsichtigkeiten ist seit Jahren ein anerkanntes und sehr effektives Verfahren zur Beseitigung von Sehfehlern. Hierbei werden inzwischen alle Sorten von Fehlsichtigkeiten wie die Kurzsichtigkeit (Myopie), die Weitsichtigkeit (Hyperopie) sowie Hornhautverkrümmungen (Astigmatismus) mit entsprechend konfektionierten Lasersystemen sehr erfolgreich behandelt. Eine Vorraussetzung für eine erfolgreiche Behandlung ist eine exakte Positionierung des Therapielaserstrahls relativ zur behandelnden Hornhaut.

Mit steigender Genauigkeit der Excimerlasersysteme sowie der Genauigkeit der zur Verfügung stehenden diagnostischen Methoden, gewinnt eine exakte Positionierung des Therapielaserstrahls am Ort der Hornhaut bzw. Cornea eine immer größer werdende Bedeutung. Seit einigen Jahren wurde die photorefraktive Korrektur der Fehlsichtigkeit von der einfachen Verwendung der Brillenwerte als Ausgangsinformationen dahingehend modifiziert, dass die räumlich aufgelösten Aberrationen des gesamten Systems des Auges mittels der Wellenfronten-Technologie sowie die topographischen Eigenschaften der Hornhaut vermessen und eine entsprechende Korrekturvorschrift für den Therapielaserstrahl erstellt wurde.

Es ist eine unmittelbare Konsequenz, dass mit der deutlichen Zunahme an Detailinformationen, die der photorefraktiven Korrektur zur Verfügung stehen, eine genaue Positionierung des zu behandelnden Auges bzw. der Behandlung am Ort der Hornhaut an Bedeutung gewinnt.

Derzeitige Therapielasersysteme werden im Allgemeinen durch sog. Blickverfolgungssysteme (Eyetracker-Systeme) unterstützt, die auf unterschiedlichsten Verfahrenstechnologien beruhen. Mit Hilfe derartiger Systeme können Positionsänderungen des Auges in der Größenordnung von unter 100 µm erkannt werden.

Eine wesentliche Vorraussetzung für eine erfolgreiche Korrektur der Fehlsichtigkeit ist somit eine genaue Orientierung und Positionierung des Therapielaserstrahls relativ zu dem zu behandelnden Auge und seiner optischen Achse, bzw. den optischen Achsen seiner refraktiv wirksamen Teilflächen. Die bisher übliche Praxis zur Justierung eines Therapielaserstrahls relativ zum Auge erfolgt bislang relativ zur Pupillenmitte oder rein subjektiv durch den behandelnden Arzt, der sich entweder an unveränderlichen Augenmerkmalen orientiert oder den Therapielaserstrahl in Abhängigkeit von an oder im Auge auftretenden Lichtreflexen manuell justiert. Allen bisher bekannten lasergestützten refraktiven Chirurgieverfahren zur optimierten Korrektur der Augenfehlsichtigkeit ermangelt es daher an einer objektiv wiederholbaren Justierung des Therapielaserstrahls relativ zu dem zu behandelnden Auge und seiner optischen Achse, bzw. den optischen Achsen seiner refraktiv wirksamen Teilflächen.

Der US 4,729,652 ist eine Vorrichtung sowie ein Verfahren zur Augenvermessung zu entnehmen, wobei die Erfassung einer Augenrotation mit Hilfe bestimmter Lichtreflexe an der Augenoberfläche, den so genannten Purkinje-Punkten vorgenommen wird.

Aus der EP 1 209 553 A1 ist ein Augenvermessungssystem zur Feststellung der Augenblickrichtung zu entnehmen, bei dem ein IR-Lichtstrahl in das Auge einfällt und teilweise an der Retina reflektiert wird. Der reflektierte Lichtanteil wir mit Hilfe einer Detektoreinheit und einer Auswerteeinheit zur Feststellung der Blickrichtung untersucht.

Schließlich ist der WO 01/45606 A2 eine Vorrichtung für die photorefraktive Keratektomie des Auges mit Zentrierung zu entnehmen, bei der die Lage des Durchstoßpunktes eines Fixierlichtstrahles durch die Kornea ermittelt wird, indem das an der Cornea durch den Fixierlichtstrahl hervorgerufene Streulicht im Durchstoßpunkt erfasst wird. Zugleich wird ein Therapierlichtstrahl koaxial zum Fixierlichtstrahl in das Auge gerichtet.

### Darstellung der Erfindung

Es besteht daher die Aufgabe eine Vorrichtung sowie ein Verfahren anzugeben, mit der ein behandelnder Arzt bei der Durchführung einer lasergestützten photorefraktiven Korrektur an einem Auge den hierfür erforderlichen Therapielaserstrahl unter ausschließlich objektiven Rahmenbedingungen relativ zum Auge automatisch einjustieren kann. So soll es möglich sein eine objektive und exakt reproduzierbare Festlegung der optimalen Position des Therapielaserstrahls zur Durchführung der Laserbehandlung an der Hornhaut zu realisieren, die unabhängig von der Arbeitsgenauigkeit des jeweiligen Operateurs ist.

Die erfindungsgemäße Vorrichtung ermöglicht es dem Arzt den Therapielaserstrahl anhand der individuellen optischen Achse des zu behandelnden Auges bzw. seiner zu behandelnden refraktiven Teilfläche auszurichten, die durch die erfindungsgemäße Vorrichtung selbsttätig bestimmbar ist.

Hierzu weist die Vorrichtung zum Erfassen der räumlichen Lage der optischen Achse des Auges eines menschlichen oder tierischen Probanden sowie zum Zentrieren eines Bezugssystems, vorzugsweise eines Therapielaserstrahls, relativ zur optischen Achse des Auges folgende Komponenten auf: - Nur der guten Ordnung halber sei an dieser Stelle darauf hingewiesen, dass die Vorrichtung auch an Tieren anwendbar ist, die weiteren Ausführungen beschränken sich jedoch, ohne den erfinderischen Gedanken einzuschränken, ausschließlich auf die Augenkorrektur bzw. die Bestimmung der optischen Achsen beim menschlichen Auge:
- Wenigstens eine, ein Parallellichtstrahlenbündel emittierende Lichtquelle, für die vorzugsweise ein im sichtbaren oder nahen infraroten Spektralbereich emittierender Laser geeignet ist, deren Lichtwellenlänge keine therapeutische Wirkung auf das Auge entfalten sollte,
- ein gegenüber der Lichtquelle vorgesehener Positionsbereich für den Probanden, so dass die Lage des zu behandelnden Auges eine weitgehend räumliche definierte Position einnimmt. Hierzu dient vorzugsweise eine an die Kopfkontur anpassbare Kopfstütze, in die der Kopf eines Probanden raumfest einlegbar ist,
- Mittel zur relativen Lageausrichtung des Parallellichtstrahlenbündels gegenüber dem Auge des Probanden. Das Mittel ist im einfachsten Fall eine als x/y-Stelltisch ausgebildete Unterlage, die zumindest in einer Ebene kontrolliert verfahrbar ist, die die Strahlrichtung des Parallellichtstrahlenbündels senkrecht schneidet, besonders bevorzugt kann vorgesehen werden den Stelltisch auch in Strahlrichtung, d.h. z-Richtung, zu bewegen, sowie
- wenigstens eine Detektoreinheit zur Detektion von in und am Auge durch das Parallellichtstrahlenbündel hervorgerufene Reflexions- und Streulichtereignisse. Für die Detektoreinheit eignet sich grundsätzlich jede Art von lichtempfindlichen Detektorsystemen, allen voran ein Bildgebendes Kamerasystem, bspw. eine Videokamera.

Erfindungsgemäß zeichnet sich die Vorrichtung dadurch aus, dass eine Auswerteeinheit vorgesehen ist, die auf der Grundlage der von der Detektoreinheit erfassten Reflexions- und Streulichtereignisse Steuersignale generiert, durch die die Mittel zur relativen Lageausrichtung angesteuert werden, wobei die Generation der Steuersignale derart erfolgt, dass wenigstens ein Reflexions- und wenigstens ein Streulichtereignis in Bezug zur Ausbreitungsrichtung des Parallellichtstrahlenbündels in Deckung zu bringen sind.

Die erfindungsgemäße Vorrichtung, die ein selbsttätiges Auffinden wenigstens eines in bzw. am Auge auftretenden Reflexions- und ein Streulichtereignis ermöglicht, bestimmt durch ein in Deckung bringen von einem am bzw. im Auge auftretenden Reflexions- und einem Streulichtereignis die visuelle bzw. optische Achse des zu behandelnden Auges bzw. eine oder mehrere seiner refraktiven Teilflächen. In Kenntnis der Raumlage der optischen Achse wird anschließend der zur Durchführung der photorefraktiven Korrektur erforderliche Therapielaserstrahl ausgerichtet.

Zum Auffinden bzw. Erfassen der optischen Achse sowie für eine während der Durchführung einer photorefraktiven Korrektur erforderliche Nachjustierung zwischen dem Therapielaserstrahl und der optischen Achse wird das Auge des Probanden relativ zum Parallellichtstrahlenbündel automatisch verfahren, so dass während des gesamten Eingriffes eine exakte Ausrichtung zwischen Auge und Lichtquelle sowie letztlich dem als Therapielaserstrahl anzusehenden Bezugssystem gewährleistet ist. Hierzu werden die von der Detektoreinheit erfassten Reflexions- und/oder Streulichtereignisse unter Verwendung einer rechnergestützten Bildverarbeitung entsprechende Steuersignale generiert, die zur Ansteuerung eines x-y-Verstelltisches dienen, auf dem der Proband aufliegt. Vorzugsweise eignet sich ein Verstelltisch, der zusätzlich in der Höhe, d.h. in z-Richtung bzw. längs der Strahlrichtung des Parallellichtstrahlenbündels beweglich ist.

Grundsätzlich ergeben sich durch das Parallellichtstrahlenbündel im oder am Auge Reflexions- und/oder Streulichtereignisse an Grenzflächen innerhalb des Auges, an denen zwei Materialschichten mit unterschiedlichen Brechungsindizes zusammentreffen.

So werden Lichtstrahlen an verschiedenen Oberflächen des Auges gebrochen und reflektiert. Eine erste Reflexion eines auf die Augenoberfläche auftreffenden Lichtstrahls tritt an der Oberfläche der Hornhaut selbst auf und wird zumeist auch als Corneareflex bezeichnet. Der Corneareflex wird in der Literatur auch als erstes Purkinje-Bild bezeichnet. Das zweite, dritte und vierte sog. Purkinje-Bild entsteht respektive an der Grenzfläche Cornea/Kammerwasser, Kammerwasser/Linse sowie Linse/Glaskörper des Auges.

Hinzukommt, dass die Hornhaut des Auges bei Durchstrahlung mit einem Parallellichtstrahlenbündel als die erste optische Komponente des Auges eine optische Wirkung auf das Strahlenbündel ausübt vergleichbar jener einer Sammellinse, die typischerweise einen Krümmungsradius in der Größenordnung von etwa 7,4 mm aufweist. Für das Auffinden der optischen. Achse des Auges müssen wenigstens zwei auf bzw. im Auge liegende, durch das Strahlenbündel hervorgerufene Lichtpunkte erkannt und zur Deckung gebracht werden.

Im Falle der Beleuchtung des Auges eines Patienten mit einem Parallelstrahlenlichtbündel, gilt es für den zu behandelnden Patienten die für ihn als Punktlichtquelle optisch in Erscheinung tretende Punktlichtquelle, von der das Parallelichstrahlenlichtbündel ausgeht, zu fixieren. Bedingt durch Reflexions- und

Streueffekte am und im Auge können bei Betrachtung des Auges folgende Lichterscheinungen bzw. Lichtpunkte festgestellt werden:
- die als Streulichtereignis in Erscheinung tretende Fixationslichtquelle,
- die Fovea Zentralis der Netzhaut, wegen der Fixation durch den Patienten, sowie
- das so genannte 1. Purkinje Bild der fixierten Lichtquelle, das als Reflexionsereignis an der Corneaoberfläche in Erscheinung tritt.

Verwendet man eine Fixationslichtquelle mit einem parallelen Strahlenlichtbündel, wie etwa eine Leuchtdiode, so kann der Durchtrittspunkt des Parallelstrahlenlichtbündels durch die Hornhaut, der an einem schwachen Streulichtreflex an der Hornhautoberfläche zu erkennen ist, über eine vergleichsweise weite Fläche der Hornhaut verfahren werden, ohne dass der Patient der sich örtlich verändernden Fixationslichtquelle nachblickt, da er wegen ihrer Parallelstrahl Eigenschaft die Lichtquelle im Unendlichen zu sehen meint und die örtliche Verschiebung der tatsächlich im Endlichen befindlichen Lichtquelle nicht erkennt. Weicht der Durchtrittspunkt des Parallestrahlenlichtbündels weiter vom Ort der optischen Achse des Auges ab, d.h. ist der Druchtrittspunkt exzentrisch, so erscheint der Streulichtreflex bzw. das Streulichtereignis der Fixationslichtquelle umso weniger hell, je exzentrischer der Durchtrittspunkt gelegen ist.

Um nun denjenigen Durchstoßungspunkt des Parallellichtstrahles durch die Hornhautoberfläche aufzufinden, welcher exakt auf der optischen Achse des Auges liegt, muss einerseits das Streulichtereignis auf der Corneaoberfläche, andererseits das als Reflexionsereignis auftretende 1. Purkinje Bild sowie die Fixationslichtquelle - unter der Bedingung zentraler Fixation durch den Patienten - in einer Flucht, d.h. längs einer gemeinsamen Raumachse liegen. Dies ist gerade dann der Fall, wenn für den betrachtenden Beobachter, d.h. für den behandelnden Arzt das 1. Purkinje-Bild koaxial mit dem Fixationslicht-Strahl ein starkes, d.h. besonders helles Reflexphänomen am Auge hervorruft. In dieser Konstellation wird das an der Corneaoberfläche am Ort des 1. Purkinje-Bildes auftreffende Parallelstrahlenlichtbündel in sich zurückreflektiert und überlagert zudem mit dem Streulichtereignis der Fixationslichtquelle. Es liegt auf der Hand, dass sich für den behandelnden Arzt auf diese Weise unverkennbar ein Lichtpunkt maximaler Lichtintensität ergibt, der eine eindeutige Navigationshilfe für die Bestimmung der Raumlage der optischen Achse des zu untersuchenden und zu behandelnden Auges des Patienten darstellt.

Gleiches kann sinngemäß für andere brechende Teilflächen des Auges zu deren Lagebestimmung angewandt werden.

Diese somit beschriebene Anordnung bietet auf Grund der vorstehend diskutierten Gegebenheiten eine praktisch ortsfeste Indikation für den Ort, an dem die optische Achse der Hornhaut unter Fixationsbedingung ihre Oberfläche "durchstößt". Dieser Durchstoßungspunkt aber ist das bestgeeignete Zentrum für eine brechkraftverändernde Krümmungsveränderung, beispielsweise mit Hilfe eines Lasers.

Auf der Grundlage dieser Eigenschaften weist die Detektoreinheit der erfindungsgemäßen Vorrichtung wenigstens ein optisches Abbildungssystem auf, durch das jeweils die im Bereich der Grenzfläche Luft/Cornea auftretenden Streulicht- und Reflexionsereignisse scharf abbildbar sind.

Durch die optische Erfassung des sog. ersten Purkinje-Bildes (Reflexionsereignis) sowie des Streulichtereignisses auf der Detektoreinheit, die vorzugsweise als Videokamera ausgebildet ist, deren Blickfeld zumindest auf den Pupillenbereich des Auges gerichtet ist und die darüber hinaus eine ortsauflösende Bildebene zur Lageerfassung der in die Bildebene der Kamera abbildbaren Reflexions- sowie Streulichtlichtereignisse vorsieht, wird unter Verwendung einer rechnergestützten Bildauswerteeinheit der Abstand sowie die Relativlagen zwischen dem Cornea-Reflex und dem Streullichtereignis ermittelt. Im Weiteren gilt es durch gezielte Lageveränderung des Auges den Cornea-Reflex und das Streulichtereignis in Deckung zu bringen. Auf der Grundlage der im Wege der Bildauswertung gewonnenen Lage- und Abstandsinformationen wird eine Trajektorie berechnet, längs der das Auge zu bewegen ist, um beide Lichtereignisse in Deckung zu bringen und letztlich in dieser Position zu verharren, zumindest solange bis eine photorefraktive Korrektur abgeschlossen ist.

Wie bereits erwähnt, erfolgt dies durch aktiv überwachte geregelte Relativbewegung des Probanden gegenüber dem Parallellichtstrahlenbündels.

Ist die optische Achse des Auges in der vorstehenden Weise erfasst und entsprechend relativ zum Parallellichtstrahlenbündel justiert, so kann zur Durchführung der photorefraktiven Korrektur ein Therapielaserstrahl längs des Parallellichtstrahlenbündels eingekoppelt werden, der durch gezielte Ablation bzw. Koagulation von Gewebebereichen im oder am Auge gewünschte Manipulationen am Auge durchführt.

### Kurze Beschreibung der Erfindung

Die Erfindung wird nachstehend unter Beschränkung des allgemeinen Erfindungsgedankens anhand eines Ausführungsbeispiels unter Bezugnahme auf die Zeichnungen exemplarisch beschrieben. Es zeigen:
- Fig. 1: Darstellung von am Auge auftretenden Reflexionsereignisse,
- Fig. 2: Beleuchtungssituation mit zentrierte optische Achse,
- Fig. 3: schematisierte Darstellung einer Vorrichtung zum automatischen Auffinden der optischen Achse durch ein Auge sowie
- Fig. 4: Strahlengeometrie zur Entstehung eines virtuellen Bildes innerhalb eines Auges und
- Fig. 5: Darstellung zur Lageveränderlichkeit des virtuellen Bildes.

### Wege zur Ausführung der Erfindung, gewerbliche Verwendbarkeit

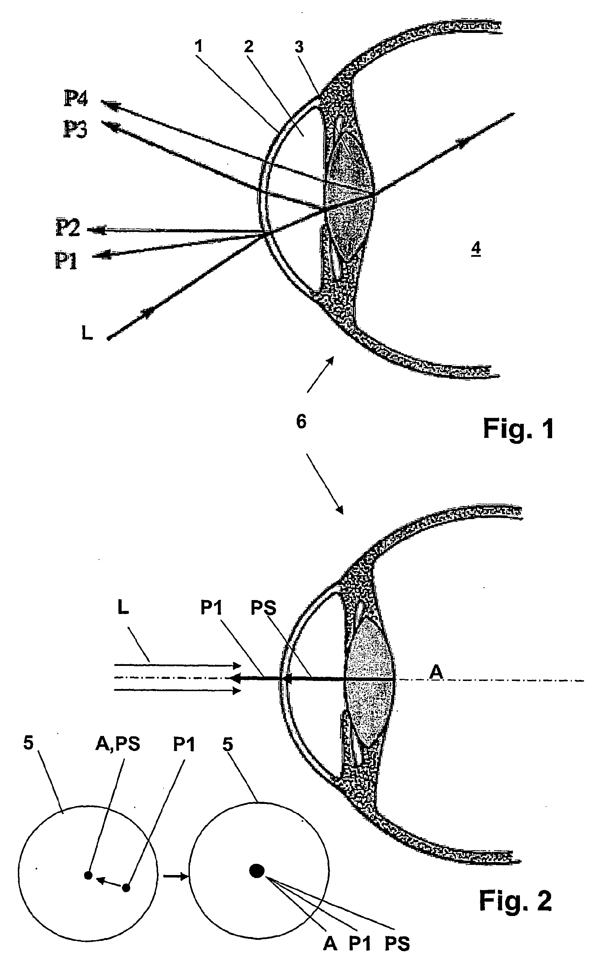

Figur 1 zeigt einen schematisierten Teilquerschnitt durch ein menschliches Auge 6, das eine Hornhaut (Cornea) 1, Kammerwasser 2, eine Linse 3 sowie den Glaskörper bzw. Augeninnenraum 4 aufweist. Trifft ein Lichtstrahl L auf die Oberfläche der

Cornea 1, so wird ein Teil des Lichtstrahles an der Grenzfläche Luft/CorneaOberfläche reflektiert. Das diesbezügliche Reflexionsereignis P1 wird als sog. Purkinje-Bild P1 oder als Cornea-Reflex bezeichnet. Ähnliche Reflexionsereignisse P2 - P4 treten an den Grenzflächen Cornea 1 /Kammerwasser 2, Kammerwasser 2 / Linse 3 sowie Linse 3 / Glaskörper 4 auf.

Zur Ermittlung der visuellen Achse A des Gesamtsystems Auge dienen im Weiteren das Reflexionsereignis P1 an der Corneaoberfläche sowie ein sich im Wege der optischen Streuung an der Corneaoberfläche ausbildendes Streulichtereignis PS, wie eingangs beschrieben.

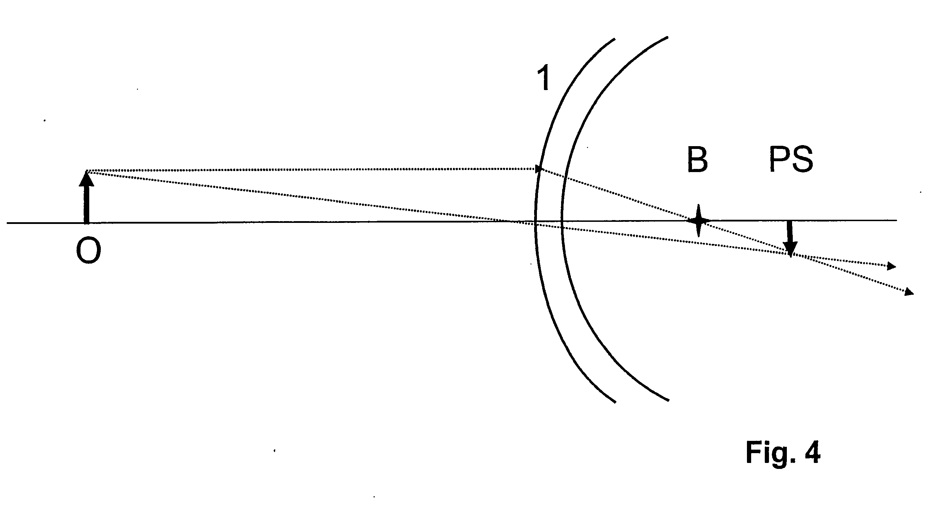

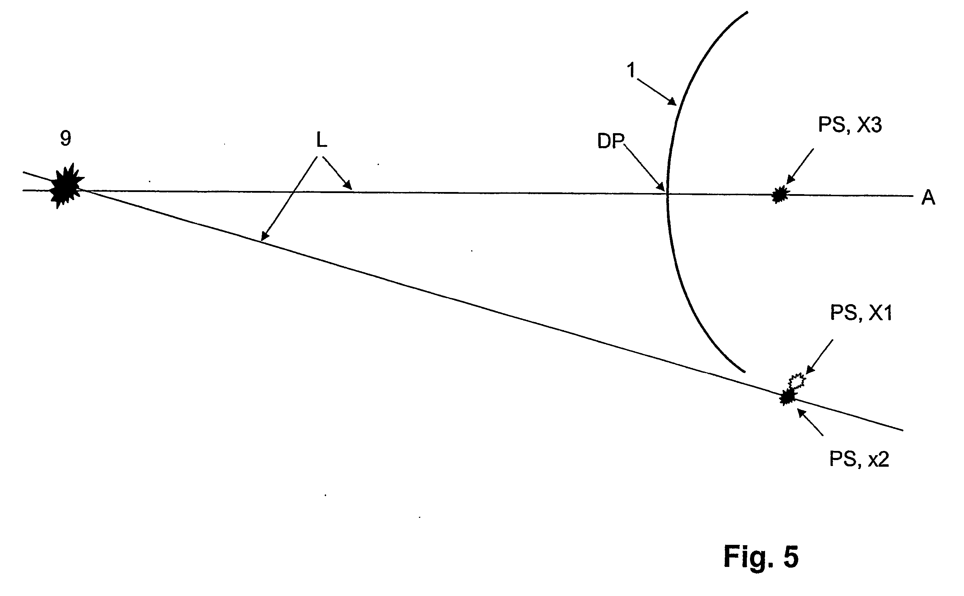

Nur aus Gründen der Vollständigkeit sei an dieser Stelle auf eine alternative Entstehungsmöglichkeit des Streulichtereignisses PS am Auge hingewiesen, die mit dem gegenwärtigen Verständnis über die optischen Gegebenheiten des Auges ebenso als denkbar angenommen werden kann und nichts an den vorstehenden Überlegungen ändert. Hierzu sei auf die Figur 4 verweisen, aus der die Strahlengeometrie einer optischen Abbildung eines Objektes O an der Cornea 1, die als Hohlspiegel wirkt und einen Hohlspiegelbrennpunkt B aufweist, hervorgeht. Das im Inneren des Auges entstehende Streulichtereignis PS kann als Abbild der Lichtquelle durch die optische Hohlspiegelwirkung aufgefasst werden und dient der exakten Bestimmung des optischen Achse A des Auges. Hierzu dient eine als punktförmige Lichtquelle 9 als Fixationstarget (siehe Figur 5), von dem ein Parallellichtstrahlenbündel L ausgeht und auf das Auge 6 des Patienten gerichtet ist. Im Falle der in der Figur 5 dargestellten Position x1 befindet sich das Streulichtereignis PS außerhalb der optischen Achse A. Die Position x2 liegt ebenfalls außerhalb der optischen Achse A, x2 soll jedoch verdeutlichen, dass die Position x2 von dem Streulichtereignis PS bei bewegtem oder verschobenen Auge eingenommen wird, d.h. die Lage des Streulichtereignisses PS ist Lageabhängig vom Auge des Patienten relativ zum Parallellichtstrahlenbündel L.

Ausschließlich im Falle, in dem das Streulichtereignis PS und der Cornea-Reftex 1 längs des Parallellichtstrahlenbündels L in Deckung sind, siehe Position x3 des Streulichtereignisses PS, ist der Durchstoßpunkt DP der optischen Achse A durch die Cornea gefunden. In diesem Fall werden Teile des Parallellichtstrahlenbündels L an der Corneaoberfläche in sich selbst zurückreflektiert und das Streulichtereignis PS gerät in Überdeckung mit diesem Reflex. Diese Konstellation zeichnet sich durch eine markante Überhellung des zu beobachtenden Reflexes am Auge aus.

Ziel ist es somit eine Beleuchtungssituation zu schaffen, bei der das auf das Auge 6 auftreffende Parallellichtstrahlenbündel L koaxial zur optischen Achse A ausgerichtet ist. Dies ist der Fall, wenn das Reflexionsereignis P1 und das Streulichtereignis PS, wie in Bilddarstellung gemäß Figur 2 dargestellt, miteinander in Deckung gebracht sind. Zur besseren Illustration sind in Figur 2 zwei kreisförmige Beobachtungsfelder 5 gezeigt, die die räumlichen Lagen von P1 und PS für eine Beleuchtung des Auges mit einem Parallellichtstrahlengang zeigen. Das linke Beobachtungsfeld stellt die Situation dar, bei der die optische Achse A des Auges bezüglich des Parallellichtstrahlenganges L dejustiert ist. In diesem Fall weicht die räumliche Lage des Reflexionsereignisses an der Corneaoberfläche P1 von der Lage des Streulichtereignisses PS, das sich bereits längs der optischen Achse A befindet, ab. Für das Auffinden der optischen Achse A, die bereits durch das Streulichtereignis PS verläuft, gilt es den Corneareflex P1 in Strahlrichtung des das Auge beleuchtenden Parallellichtstrahlenbündels L mit dem Streulichtereignis PS in Deckung zu bringen. Dies ist in der rechten Kreisbilddarstellung gemäß Figur 2 dargestellt. Befinden sich beide Lichtereignisse P1 und PS in Deckung, so fällt ihre Verbindungsachse mit der visuellen bzw. optischen Achse A des Auges zusammen, wodurch die räumliche Lage des Auges erfasst und festgelegt ist.

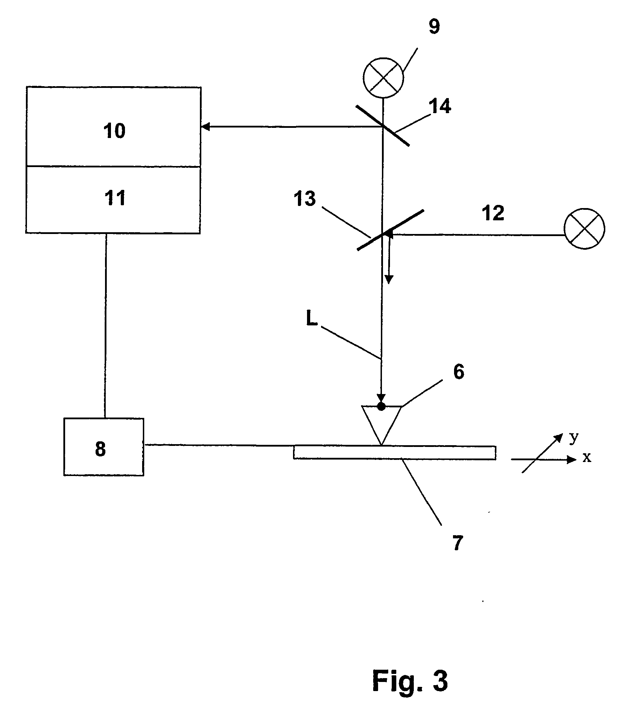

Die in Figur 3 schematisch dargestellte Vorrichtung vermag nun einerseits die optische Achse des Auges 6 automatisch zu ermitteln und überdies die erfasste optische Achse des Auges 6 in Bezug zu einem optischen Bezugssystems, bspw. zu einem Therapielaserstrahl entsprechend nachzuführen bzw. zu fixieren. Hierzu wird das zu behandelnden Auge 6 eines nicht weiter dargestellten Probandens, der auf einer Patientenliege 7 liegt, die in der x-y-Ebene um wenigstens zwei Raumachsen über motorisch angetriebene Stellmittel 8 verfahrbar ist, positioniert. Eine Lichtquelle 9 sendet zur Erzeugung der vorstehend beschriebenen Reflexions- und Streulichtereignisse am und im Auge 6 ein Parallellichtstrahlenbündel L aus, das in den Pupillenbereich des Auges 6 gerichtet ist. Über eine nicht weiter dargestellte Abbildurigsoptik werden die am oder im Auge auftretenden Reflexions- und Streulichtereignisse in eine Detektoreinheit 10 über eine Umlenkeinheit 14 abgebildet, in der sich die in der Figur 2 dargestellten Blickfelder 5 ergeben. Die
Detektoreinheit 10 ist vorzugsweise als Videokamera ausgebildet und dient der ortsauflösenden Lageerfassung der in der Bildebene der Videokamera abgebildeten Reflexions- und Streulichtereignisse hinsichtlich des Purkinje-Bildes P1 und des Streulichtereignisses PV, wie vorstehend erläutert. Vorzugsweise könnte zur Erfassung der beiden Lichtereignisse auch jeweils ein getrenntes Abbildungssystem bzw. Kamerasystem eingesetzt werden, um auf diese Weise beide voneinander in gegebenenfalls in der Tiefe zueinander beabstandete Lichtereignisse scharf abzubilden.

Mit Hilfe einer rechnergestützten graphischen Bildauswerteeinheit 11 gilt es die Lichtereignisse P1 und PS relativ zur Strahlrichtung des Parallellichtstrahlenbündels L miteinander in Deckung zu bringen. Hierzu wird eine Trajektorie ermittelt, mit der die zunächst räumlich auseinander liegenden Lichtereignisse P1 und PS miteinander in Deckung gebracht werden können. Die Auswerteeinheit 11 erzeugt hierzu Steuersignale, die an die Verstelleinheit 8 übertragen werden, wodurch die Patientenliege in entsprechende Position gebracht wird. Der Vorgang des in Deckung bringen der beiden innerhalb des Auges 6 entstehenden Lichtereignisse P1 und PS erfolgt vollautomatisch. Sobald sich die Lichtereignisse P1 und PS in Strahlrichtung des Parallellichtstrahlenbündels L in Deckung befinden, definiert ihre Verbindungsachse die visuelle bzw. optische Achse des Auges 6. In eben dieser Konfiguration können weiterführende optische Manipulationen am Auge durchgeführt werden. Bspw. eignet sich die Einkopplung eines Therapielaserstrahls 12 in den Strahlengang des Parallellichtstrahlenbündels L mit Hilfe eines halbdurchlässigen Umlenkspiegels 13. Der Therapielaserstrahl 12 dient vorzugsweise zur Durchführung photorefraktiver Korrekturmaßnahmen in oder am Auge. Sollte sich die Lage der optischen Achse in Bezug zum Parallellichtstrahlenbündel sowie auch zum Therapielaserstrahl während der Behandlung ändern, so wird eine Dejustierung aufgrund der sich ändernden Positionen der jeweiligen Lichtereignisse P1 und PS durch die Detektoreinheit 10 erfasst und unter Echtzeitbedingungen entsprechende Korrektursteuersignale zur Nachjustierung des Auges erzeugt.

Die in Figur 3 schematisiert dargestellte erfindungsgemäße Vorrichtung dient somit dem vollautomatischen Auffinden der optischen Achse eines Auges sowie deren räumliche Fixation im Wege einer geregelten Justierung bzw. Nachführung des Auges relativ zu einem optischen Bezugssystem zur Durchführung insbesondere photorefraktiver Korrekturmaßnahmen am Auge.

### Bezugszeichenliste

- 1: Cornea
- 2: Kammerwasser
- 3: Linse
- 4: Glaskörper
- 5: Blickfeld
- 6: Auge
- 7: Positionsbereich, Patientenliege
- 8: Verstelleinheit
- 9: Lichtquelle
- 10: Detektoreinheit
- 11: Auswerteeinheit
- 12: Therapielaserstrahl
- 13: Umlenkspiegel
- 14: Umlenkspiegel
- L: Parallellichtstrahlenbündel
- DP: Durchstoßpunkt
- A: Optische Achse
- B: Brennpunkt der Hornhaut
- PS: Streulichtereignis

## Patentansprüche

1. Vorrichtung zum Erfassen der räumlichen Lage der optischen Achse (A) des Auges (6) eines menschlichen oder tierischen Probanden sowie zum Zentrieren eines Bezugssystems relativ zur optischen Achse (A), mit wenigstens einer ein Parallellichtstrahlenbündel (L) emittierenden Lichtquelle (9), einem gegenüber der Lichtquelle (9) vorgesehenen Positionsbereich (7) für den Probanden, Mittel zur relativen Lageausrichtung des Parallellichtstrahlenbündels (L) gegenüber dem Auge (6) des Probanden sowie wenigstens einer Detektoreinheit (10) zur Detektion von in und am Auge (6) durch das Parallellichtstrahlenbündel (L) hervorgerufene Reflexionsereignissen,
**dadurch gekennzeichnet, dass** eine Auswerteeinheit (11) vorgesehen ist, die auf der Grundlage der von der Detektoreinheit (10) erfassten Streu- und Reflexionsereignissen Steuersignale generiert, durch die die Mittel zur relativen Lageausrichtung angesteuert werden, wobei die Generation der Steuersignal derart erfolgt, dass wenigstens ein Reflexions- und wenigstens ein Streulichtereignis (PS) in Bezug zur Ausbreitungsrichtung des Parallellichtstrahlenbündels (L) in Deckung zu bringen sind.

2. Vorrichtung nach Anspruch 1,
**dadurch gekennzeichnet, dass** das Auge (6) in Durchstrahlungsrichtung des Parallellichtstrahlenbündels (L) wenigstens folgende vier optisch wirksame Grenzflächen aufweist: Luft/Hornhaut, Hornhaut/Kammerwasser, Kammerwasser/Linse und Linse/Glaskörper,
dass die Detektoreinheit (10) wenigstens ein optisches Abbildungssystem aufweist, durch das jeweils ein im Bereich der Grenzfläche Luft/Hornhaut auftretendes Reflexionsereignis (P1), so genanntes erstes Purkinje-Bild, sowie ein am Auge (6) auftretendes Streulichtereignis (PS) scharf abbildbar sind.

3. Vorrichtung nach Anspruch 1 oder 2,
**dadurch gekennzeichnet, dass** die Detektoreinheit (10) wenigstens eine Kamera, vorzugsweise wenigstens eine Videokamera, vorsieht
mit einem zumindest auf den Pupillenbereich des Auges (6) gerichteten Blickfeld und einer ortsauflösenden Bildebene zur Lageerfassung der in die Bildebene der Videokamera abbildbaren Reflexions- (P1) und/oder Streulichtereignissen (PS).

4. Vorrichtung nach einem der Ansprüche 1 bis 3,
**dadurch gekennzeichnet, dass** die Mittel zur relativen Lageausrichtung des Parallelstrahlenlichtbündels (L) gegenüber dem Auge (6) des Probanden wenigstens eine Verstelleinheit (8) vorsehen, durch die der für den Probanden vorgesehene Positionsbereich (7) gegenüber der stationären Lichtquelle (9) in wenigstens einer Ebene bewegbar ist.

5. Vorrichtung nach einem der Ansprüche 1 bis 4,
**dadurch gekennzeichnet, dass** die Auswerteeinheit (11) eine rechnergestützte graphische Bildauswerteeinheit vorsieht, die automatisch wenigstens das erste Purkinje Bild (P1) und das Streulichtereignis (PS) erfasst und eine Trajektorie berechnet um durch relative Lageveränderung zwischen Lichtquelle (9) und Auge (6) des Probanden beide Lichtereignisse in Deckung zu bringen und zu halten.

6. Vorrichtung nach einem der Ansprüche1 bis 5,
**dadurch gekennzeichnet, dass** eine weitere Lichtquelle vorgesehen ist, deren Lichtstrahl über wenigstens ein optisches Umlenkelement (13) in den auf das Auge (6) gerichteten Parallellichtstratil (L) einkoppelbar ist.

7. Vorrichtung nach Anspruch 6,
**dadurch gekennzeichnet, dass** die weitere Lichtquelle ein Therapielaser (12) zur gezielten Ablation und/oder Koagulation von Gewebebereichen am oder im Auge (6) ist.

8. Vorrichtung nach Anspruch 6 oder 7,
**dadurch gekennzeichnet, dass** der Lichtstrahl der weiteren Lichtquelle (12) und/oder das Parallellichtstrahlenbündel (L) das Bezugssystem darstellt

9. Verfahren zum Erfassen der räumlichen Lage der optischen Achse (A) eines Auges (6) eines menschlichen oder tierischen Probanden sowie zum Zentrieren eines Bezugssystems relativ zur optischen Achse (A),
bei dem das Auge (6) mit wenigstens einem Parallellichtstrahlenbündel (L) beleuchtet wird, das Auge (6) des Probanden und/oder das Parallellichtstrahlenbündel (L) relativ zueinander lagerveränderlich gelagert wird und mittels einer Detektoreinheit (10) an und/oder innerhalb des Auges (6) durch das Parallellichtstrahlenbündel (L) hervorgerufene Reflexionsereignisse erfasst werden, **dadurch gekennzeichnet, dass** ein an der Hornhautoberfläche durch das Parallellichtstrahlenbündel (L) hervorgerufenes Reflexionsereignis (P1) sowie ein am Auge (6) hervorgerufenes Streulichtereignis (PS) mit der Detektoreinheit (10) erfasst werden, und
dass das Auge (6) des Probanden relativ zum Parallellichtstrahlenbündel (L) derart Lage verändert wird, dass längs des Parallellichtstrahlenbündels (L) das Reflexionsereignis (P1) an der Hornhautoberfläche und das Streulichtereignis (PS) in Deckung gebracht und gehalten werden.

10. Verfahren nach Anspruch 9,
**dadurch gekennzeichnet, dass** im Falle der axialen Koinzidenz zwischen dem an der Hornhautoberfläche auftretenden Reflexionsereignis (P1) und dem Streulichtereignis (PS), der mit dem Reflexionsereignis (P1) verbundene Reflexionsort als Durchstoßpunkt (DP) der optischen Achse (A) des Auges (6) durch die Hornhaut bestimmt wird.

11. Verfahren nach Anspruch 10,
**dadurch gekennzeichnet, dass** die räumliche Lage des Durchstoßpunktes (DP) abgespeichert wird.

## Claims

1. A device for detecting the spatial position of the optical axis (A) of the eye (6) of a human or animal subject, and for centring a reference system in relation to the optical axis (A), having at least one light source (9) emitting a parallel light beam bundle (L), a positioning region (7) for the subject provided opposite the light source (9), means for the relative position orientation of the parallel light beam bundle (L) in relation to the eye (6) of the subject, and at least one detector unit (10) for detecting reflection events caused in and on the eye (6) by the parallel light beam bundle (L),
**characterised in that** an analysis unit (11) is provided, which generates control signals on the basis of the scattering and reflection events detected by the detector unit (10), by which the means for relative position orientation are activated, wherein
the control signals are generated in such a way that at least one reflection event and at least one scattered light event (PS) are to be brought into congruency in relation to the propagation direction of the parallel light beam bundle (L).

2. The device according to Claim 1,
**characterised in that** the eye (6) has at least the following four optically active interfaces in the irradiation direction of the parallel light beam (L):
air/cornea, cornea/aqueous humour, aqueous humour/lens, and lens/vitreous humour, and
**in that** the detector unit (10) has at least one optical imaging system, by which a reflection event (P1) occurring in the area of the air/cornea interface, known as the first Purkinje image, and a scattered light event (PS) occurring on the eye (6), can be imaged sharply.

3. The device according to Claim 1 or 2,
**characterised in that** the detector unit (10) provides at least one camera, preferably at least one video camera,
having at least one field of vision directed to the pupil area of the eye (6) and a position-resolving image plane for position detection of the reflection light events (P1) and/or scattered light events (PS) that can be imaged in the image plane of the video camera.

4. The device according to one of the Claims 1 to 3,
**characterised in that** the means for relative position orientation of the parallel light beam bundle (L) in relation to the eye (6) of the subject provide at least one adjustment unit (8), by which the positioning region (7) provided for the subject is movable in relation to the stationary light source (9) in at least one plane.

5. The device according to one of the Claims 1 to 4,
**characterised in that** the analysis unit (11) provides a computer-supported graphic image analysis unit, which automatically detects at least the first Purkinje image (P1) and the scattered light event (PS), and calculates a trajectory to bring and keep the two light events in congruency by a relative change in position between light source (9) and eye (6) of the subject.

6. The device according to one of the Claims 1 to 5,
**characterised in that** a further light source is provided, whose light beam can be coupled into the parallel light beam (L) directed onto the eye (6) via at least one optical deflection element (13).

7. The device according to Claim 6,
**characterised in that** the further light source is a treatment laser (12) for targeted ablation and/or coagulation of tissue areas on or in the eye (6).

8. The device according to Claim 6 or 7,
**characterised in that** the light beam of the further light source (12) and/or the parallel light beam bundle (L) represents the reference system.

9. A method for detecting the spatial position of the optical axis (A) of an eye (6) of a human or animal subject and for centring a reference system in relation to the optical axis (A),
in which the eye (6) is illuminated using at least one parallel light beam bundle (L), the eye (6) of the subject and/or the parallel light beam bundle (L) are mounted in relation to one another such that their positions may be changed, and reflection events caused by the parallel light beam bundle (L) on and/or inside the eye (6) are detected using a detector unit (10),
**characterised in that** a reflection event (P1) caused by the parallel light beam bundle (L) on the cornea surface and also a scattered light event (PS) caused on the eye (6) are detected using the detector unit (10), and
**in that** the eye (6) of the subject is changed in its position in relation to the parallel light beam bundle (L) in such a way that the reflection event (P1) on the cornea surface and the scattered light event (PS) are brought into congruency along the parallel light beam bundle (L) and held there.

10. The method according to Claim 9,
**characterised in that**, in the case of axial coincidence between the reflection event (P1) occurring on the cornea surface and the scattered light event (PS), the reflection position connected to the reflection event (P1) is determined as the penetration point (DP) of the optical axis (A) of the eye (6) through the cornea.

11. The method according to Claim 10,
**characterised in that** the spatial position of the penetration point (DP) is stored.

## Revendications

1. Dispositif pour détecter la position spatiale de l'axe optique (A) de l'oeil (6) d'un sujet humain ou animal, ainsi que pour centrer un système de référence par rapport à l'axe optique (A), avec au moins une source de lumière (9) émettant un faisceau de rayons de lumière parallèles (L), une zone de positionnement (7) pour le sujet, prévue face à la source de lumière (9), des moyens pour un alignement relatif du faisceau de rayons de lumière parallèles (L) par rapport à l'oeil (6) du sujet, ainsi qu'au moins une unité de détection (10) pour détecter des événements de réflexion provoqués dans et au niveau de l'oeil (6) par le faisceau de rayons de lumière parallèles (L),
**caractérisé en ce qu'**une unité d'analyse (11) est prévue qui génère des signaux de commande sur la base des événements de dispersion et de réflexion détectés par l'unité de détection (10), lesdits signaux pilotant les moyens pour l'alignement relatif, dans lequel la génération du signal de commande est effectuée de telle sorte qu'il s'agit de faire converger au moins un événement de réflexion et au moins un événement de lumière diffusée (PS) par rapport à la direction de propagation du faisceau de rayons de lumière parallèles (L).

2. Dispositif selon la revendication 1,
**caractérisé en ce que** l'oeil (6) dans la direction de transmission du faisceau de rayons de lumière parallèles (L) présente au moins quatre interfaces optiquement actives : air/cornée, cornée/humeur aqueuse, humeur aqueuse/lentille et lentille/corps vitré,
**en ce que** l'unité de détection (10) présente au moins un système de reproduction optique permettant de reproduire de façon nette, respectivement un événement de réflexion (P1) apparaissant dans la zone de l'interface air/cornée, une première image dite de Purkinje, ainsi qu'un événement de lumière diffuse (PS) apparaissant au niveau de l'oeil (6).

3. Dispositif selon la revendication 1 ou 2,
**caractérisé en ce que** l'unité de détection (10) prévoit au moins une caméra, de préférence au moins une caméra vidéo,
avec un champ de vision dirigé au moins vers la zone de pupille de l'oeil (6) et un plan image à résolution locale pour la détection de position des événements de réflexion (P1) et/ou de lumière diffuse (PS) reproductibles dans le plan image de la caméra vidéo.

4. Dispositif selon l'une quelconque des revendications 1 à 3,
**caractérisé en ce que** les moyens pour l'alignement relatif du faisceau de rayons de lumière parallèles (L) par rapport à l'oeil (6) du sujet prévoient au moins une unité de réglage (8) qui permet de déplacer la zone de positionnement (7) prévue pour le sujet par rapport à la source de lumière (9) stationnaire, dans au moins un plan.

5. Dispositif selon l'une quelconque des revendications 1 à 4,
**caractérisé en ce que** l'unité d'analyse (11) prévoit une unité d'analyse d'image graphique assistée par ordinateur qui détecte automatiquement au moins la première image de Purkinje (P1) et l'événement de lumière diffuse (PS) et calcule une trajectoire pour faire converger et maintenir les deux événements lumineux par un changement de position relatif entre la source de lumière (9) et l'oeil (6) du sujet.

6. Dispositif selon l'une quelconque des revendications 1 à 5,
**caractérisé en ce qu'**une autre source de lumière est prévue dont le faisceau lumineux peut être injecté par l'intermédiaire d'au moins un élément de déviation optique (13) dans le rayon de lumière parallèle (L) dirigé sur l'oeil (6).

7. Dispositif selon la revendication 6,
**caractérisé en ce que** l'autre source de lumière est un laser thérapeutique (12) pour l'ablation et/ou la coagulation ciblée de zones de tissu au niveau de ou dans l'oeil (6).

8. Dispositif selon la revendication 6 ou 7,
**caractérisé en ce que** le faisceau lumineux de l'autre source de lumière (12) et/ou le faisceau de rayons de lumière parallèles (L) représente le système de référence.

9. Procédé pour détecter la position spatiale de l'axe optique (A) d'un oeil (6) d'un sujet humain ou animal, ainsi que pour centrer un système de référence par rapport à l'axe optique (A),
dans lequel l'oeil (6) est éclairé par au moins un faisceau de rayons de lumière parallèles (L), l'oeil (6) du sujet et/ou le faisceau de rayons de lumière parallèles (L) sont situés l'un par rapport à l'autre avec une possibilité de changement de position, et des événements de réflexion, provoqués par le faisceau de rayons de lumière parallèles (L), sont détectés au moyen d'une unité de détection (10) au niveau et/ou à l' intérieur de l'oeil (6),
**caractérisé en ce qu'**un événement de réflexion (P1) provoqué par le faisceau de rayons de lumière parallèles (L) à la surface de la cornée, ainsi qu'un événement de lumière diffuse (PS) provoqué au niveau de l'oeil (6), sont détectés par l'unité de détection (10), et
**en ce que** la position de l'oeil (6) du sujet par rapport au faisceau de rayons de lumière parallèles (L) est modifiée de telle sorte que le long du faisceau de rayons de lumière parallèles (L), l'événement de réflexion (P1) au niveau de la surface de la cornée et l'élément de lumière diffuse (PS) sont amenés à converger et sont maintenus.

10. Procédé selon la revendication 9,
**caractérisé en ce qu'**en cas de coïncidence axiale entre l'événement de réflexion (P1) apparaissant au niveau de la surface de la cornée et l'événement de lumière diffuse (PS), le lieu de réflexion associé à l'événement de réflexion (P1) est déterminé comme point de percée (DP) de l'axe optique (A) de l'oeil (6) à travers la cornée.

11. Procédé selon la revendication 10,
**caractérisé en ce que** la position spatiale du point de percée (DP) est mémorisée.
